(19) Europäisches Patentamt
European Patent Office
Office européen des brevets

(11) Veröffentlichungsnummer: **0 333 142**
**A2**

(12) **EUROPÄISCHE PATENTANMELDUNG**

(21) Anmeldenummer: **89104541.1**

(51) Int. Cl.4: **C12P 41/00 , C12P 17/02**

(22) Anmeldetag: **14.03.89**

(30) Priorität: **17.03.88 CH 1013/88**

(43) Veröffentlichungstag der Anmeldung:
**20.09.89 Patentblatt 89/38**

(84) Benannte Vertragsstaaten:
**AT BE CH DE FR GB IT LI NL SE**

(71) Anmelder: **LONZA AG**
**Gampel/Wallis Geschäftsleitung Basel**
**CH-4002 Basel(CH)**

(72) Erfinder: **Günther, Joachim, Dr., Dipl.-Chem.**
**Gestaltenrainweg 63**
**Riehen (Kanton Basel-Stadt)(CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-8000 München 40(DE)**

(54) **Verfahren zur Herstellung von (R)-Glycidylestern.**

(57) Verfahren zur Herstellung von (R)-Glycidylestern niedriger Carbonsäuren aus den entsprechenden Rasematen durch enantioselektive Hydrolyse der (S)-Enantiomeren mittels Lipasen, vorzugsweise Schweinepankreas-Lipase, in Gegenwart von Fettsäuren oder deren wasserlöslichen Salzen.

EP 0 333 142 A2

## Verfahren zur Herstellung von (R)-Glycidylestern

Unter den chiralen $C_3$-Bausteinen für die organische Synthese spielen unsymmetrisch substituierte Glycerinderivate eine bedeutende Rolle. Besonders vielseitig verwendbar sind die Derivate des Glycidols, von denen das (R)-Glycidylbutyrat (I, R = butyryl) und das (R)-Glycidyltosylat (I, R = p-toluolsulfonyl) als Ester des (S)-Glycidols (I, R = H) mit Buttersäure bzw. p-Toluolsulfonsäure bereits kommerziell erhältlich sind.

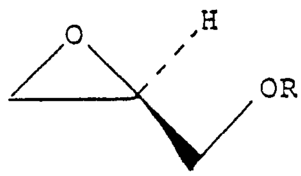

(I)

In der Literatur sind speziell für das (R)-Glycidylbutyrat zahlreiche Anwendungen beschrieben. Diese umfassen sowohl die Umwandlung in andere chirale Synthesebausteine, wie (S)-2,3-O-Isopropylidenglycerinaldehyd [S.K.Kang und D.S.Shin, Bull. Korean Chem.Soc. 7, 159 (1987)], (S)-1-Brom-2,3-propandiol S.Y.Ko, H.Masamune und K.B.Sharpless, J.Org.Chem. 52, 667 (1987)], (R)-1,2-Propylenglykol [A.Kötz und K.Richter, J.Prakt. Chem. [2] 111, 397 (1925)] oder chirale Acylglycidylether D.E.McClure, B.H.Harrison und J.J.Baldwin, J.Am.Chem.Soc. 101 3666 (1979)], als auch die Herstellung von β-Blockern [D.E.McClure, B.H.Harrison und J.J.Baldwin, loc.cit.] oder von chiralen Glyceriden [C.A.Lok, J.P.Ward und D.A.v.Dorp, Chem.Phys. Lipids 16, 115 (1976)].

Für alle diese Anwendungen ist (R)-Glycidylbutyrat natürlich umso wertvoller, je höher seine optische Reinheit (angegeben als "enantiomeric excess", abgekürzt ee) ist, weil bei genügender optischer Reinheit auf eine gesonderte Abtrennung der unerwünschten Enantiomeren aus den Produkten verzichtet werden kann.

Zweckmässig sind Werte im Bereich von grösser als 95% ee, also ein Anteil von kleiner als 2,5% des unerwünschten Enantiomeren. Die bisher im Handel erhältlichen Präparate von (R)-Glycidylbutyrat weisen dagegen meist nur Werte von ca. 85 bis 90% ee auf.

(RS)-Glycidylester niedriger Carbonsäuren sind aus (RS)-Glycidol und Acylchloriden oder aus (RS)-Epichlorhydrin und Alkalicarboxylaten, also aus billigen Ausgangsmaterialien, nach bekannten Verfahren kostengünstig herzustellen.

Verfahren zur Herstellung von optisch reinen Glycidylestern niedriger Carbonsäuren gehen daher zweckmässig von den entsprechenden (RS)-Glycidylestern aus und unterziehen diese einer Enantiomerentrennung. Die klassischen Methoden zur Enantiomerentrennung erfordern jedoch in der Regel teure chirale Hilfsstoffe in stöchiometrischen Mengen und mehrere Verfahrensschritte.

Ein neueres Verfahren zur Herstellung von (R)-Glycidylestern [W.E.Ladner und G.M.Whitesides, J.Am.Chem.Soc. 106, 7250 (1984)] macht dagegen Gebrauch von der enantioselektiven enzymatischen Hydrolyse der (RS)-Glycidylester mit Schweinepankreas-Lipase, bei der bevorzugt das (S)-Enantiomere zu (R)-Glycidol und Carboxylat hydrolysiert wird, während das (R)-Enantiomere sich im Rückstand anreichert und schliesslich aus diesem isoliert wird. Unter idealen Verhältnissen sollte so aus einem vollständig racemischen Gemisch nach einem Umsatz von genau 50%, nämlich gerade dem Anteil des (S)-Enantiomeren, der gesamte Anteil des (R)-Enantiomeren in einer optischen Reinheit von 100% ee erhalten werden. Die bisher erzielten praktischen Resultate weichen jedoch deutlich von diesen Werten ab. Es zeigte sich, dass das Maximum der optischen Reinheit des unumgesetzten Anteils an Glycidylester erst nach einem Umsatz von grösser als 50% erreicht wird, bzw. dass die optische Reinheit mit weiter zunehmendem Umsatz allmählich immer weiter ansteigt. Die bei einem Umsatz von 60% erreichten Werte liegen maximal bei 90% ee. Dieser Reinheitsgrad ist einerseits für viele Anwendungen noch nicht ausreichend, andererseits ist es bei einem technischen Verfahren unbefriedigend, um einer geringfügig höheren Produktereinheit willen eine wesentlich schlechtere Ausbeute in Kauf zu nehmen, was die Folge einer bis zu höherem Umsatz durchgeführten Hydrolyse wäre.

Der Erfindung liegt daher die Aufgabe zugrunde, (R)-Glycidylester niedriger Carbonsäuren, insbesondere (R)-Glycidylbutyrat, in hoher optischer Reinheit kostengünstig herzustellen.

Es wurde nun gefunden, dass die optische Reinheit des bei der enzymatischen Hydrolyse des in (RS)-Glycidylestern niedriger Carbonsäuren enthaltenen (S)-Enantiomeren zurückbleibenden (R)-Enantiomeren wesentlich verbessert werden kann, indem bei der Hydrolyse Fettsäuren oder deren wasserlösliche Salze in

Konzentrationen von 0,01 bis 2,0 mol/l zugesetzt werden.

Die nach dem erfindungsgemässen Verfahren herstellbaren (R)-Glycidylester sind die Glycidylester von n-Carbonsäuren mit 2 bis 6 C-Atomen, also Essigsäure, Propionsäure, Buttersäure, Valeriansäure und Capronsäure, vorzugsweise Buttersäure.

Als Enzyme werden Schweinepankreas-Lipase, Mucor-Lipase (aus Mucor miehei) oder Amano-P-Lipase (Amano Pharmaceutical Co.Ltd. Nagoya 460 Japan) (aus Pseudomonas sp.) eingesetzt, vorzugsweise jedoch rohe Schweinepankreas-Lipase.

Als zugesetzte Fettsäuren finden zweckmässig gesättigte oder ungesättigte Monocarbonsäuren mit 4 bis 24 C-Atomen, wie z.B. Buttersäure, Capronsäure, Heptansäure, Caprylsäure, Pelargonsäure, Caprinsäure, Laurinsäure, Palmitinsäure, Stearinsäure, Oelsäure, Linolsäure, Arachinsäure, Behensäure oder deren wasserlösliche Salze, wie Natrium- oder Kaliumsalze, einzeln oder in Mischungen Verwendung, vorzugsweise jedoch rohe Oelsäure.

Die Hydrolyse wird vorzugsweise in einem Zweiphasensystem, bestehend aus einer Glycidylester-Phase und einer wässrigen Phase, welche das Enzym, die Hydrolyseprodukte (R)-Glycidol und Carbonsäuresalz, sowie die zugesetzte Fettsäure bzw. deren wasserlösliches Salz enthält, unter Rühren und ohne zusätzliches Lösungsmittel durchgeführt.

Die angegebenen Konzentrationen der erfindungsgemäss zugesetzten Fettsäuren oder wasserlöslichen Fettsäuresalze beziehen sich in diesem Fall auf das gesamte Reaktionssystem, wobei sich die tatsächlichen Konzentrationen in den beiden Phasen aufgrund der jeweiligen Verteilungskoeffizienten bei den angegebenen pH-Werten einstellen.

Die Temperatur bei der Hydrolyse beträgt zweckmässig 0 bis 30 °C, vorzugsweise 0 bis 10 °C, sie wird zweckmässig durch einen Thermostaten konstant gehalten.

Der pH-Wert der wässrigen Phase liegt während der Hydrolyse zweckmässig im Bereich pH 5 bis pH 8, vorzugsweise bei 5,8 bis 6,5, er wird vorzugsweise mit Hilfe einer geeigneten Vorrichtung ("pH-Stat"), zweckmässig durch kontinuierliche Zugabe einer Base, vorzugsweise wässriger Natriumhydroxidlösung, konstant gehalten, wobei vorzugsweise die zugegebene Basenmenge in Abhängigkeit von der Reaktionsdauer durch eine Schreibvorrichtung aufgezeichnet wird.

Zweckmässig wird die Hydrolyse bis zu einem Umsatz von 50 bis 70%, d.h. bis 50 bis 70% der gesamten Glycidylestermenge hydrolysiert sind, fortgesetzt und dann abgebrochen, beispielsweise indem der nicht umgesetzte Anteil, also der (R)-Glycidylester, in einem geeigneten Lösungsmittel, wie z.B. Dichlormethan, aufgenommen und von der wässrigen Phase getrennt wird. Vorzugsweise erfolgt der Abbruch der Hydrolyse nach Erreichen eines Umsatzes von 55 bis 65%.

## Beispiele

Zur Beurteilung der Wirksamkeit von Fettsäurezusätzen wurden die Enantioselektivitäten der enzymatischen Hydrolyse jeweils mit und ohne Zusatz unter sonst identischen Bedingungen bestimmt. Dazu wurde jeweils die Reaktionsgeschwindigkeit der Hydrolyse von (RS)-Glycidylbutyrat und von reinem (R)-Glycidylbutyrat unter identischen Bedingungen zu Beginn der Reaktion bestimmt und daraus der Quotient Q gemäss folgender Gleichung gebildet:

$$Q = \frac{A - B}{B}$$

wobei A = Reaktionsgeschwindigkeit mit (RS)-Glycidylbutyrat und B = Reaktionsgeschwindigkeit mit (R)-Glycidylbutyrat. Dieser Quotient ist ein direktes Mass für die Enantioselektivität der Hydrolyse, sein Wert beträgt Null für eine nicht enantioselektive Reaktion und wird unendlich für eine vollständig enantioselektive Reaktion. Er ist einfach zu bestimmen, da die Reaktionsgeschwindigkeiten bei der gewählten Versuchsanordnung unmittelbar aus der Steigung der Titrationskurven des pH-Staten abgelesen werden können und nur ein Enantiomeres in reiner Form zur Verfügung stehen muss. Die Bestimmung der optischen Reinheit des Produktes ist dagegen aufwendiger, da sie in der Regel erst nach der Aufarbeitung vorgenommen werden kann und (bei polarimetrischer Bestimmung) im hier interessierenden Bereich von grösser als 90% ee auch zu ungenau ist.

Beispiel 1

In einem 1000 ml-Reaktionsgefäss mit Kühlmantel wurden 100 g (RS)-Glycidylbutyrat, 400 ml entsalztes Wasser und 6,25 g Oelsäure (technisch, 70%) unter Rühren auf -0,5° C gekühlt und mit 2 N Natronlauge auf pH 6,0 eingestellt. Dann wurden 0,5 g Schweinepankreas-Lipase (3-4 U/mg, Fluka) zugefügt und der pH-Wert mit 2 N NaOH mittels eines pH-Staten auf 5,9 bis 6,0 gehalten.
Nach ca. 24 h Rühren bei -0,5° C wurde die Reaktion bei einem Umsatz von 57% abgebrochen, indem der pH-Wert auf 7,0 eingestellt und der Reaktionsansatz mit 400 ml Dichlormethan ausgeschüttelt wurde.
Nach Abtrennung der organischen Phase durch Zentrifugieren wurde die wässrige Phase noch zweimal mit je 100 ml Dichlormethan ausgeschüttelt. Die vereinigten organischen Phasen wurden mit 50 ml 10% wässriger Natriumhydrogencarbonatlösung und zweimal mit je 50 ml Wasser gewaschen und anschliessend mit 2 g Magnesiumsulfat und 0,2 g Natriumcarbonat getrocknet. Nach Abdestillieren des Dichlormethans bei 300 mbar wurde das Rohprodukt (ca. 60 g) bei 6 bis 7 mbar über eine Vigreux-Kolonne (l = 15 cm) fraktioniert. Nach einem Vorlauf von 2,2 g ging bei 65° C Kopftemperatur die Hauptfraktion über.
Ausbeute: 33,6 g (67,2%, bezogen auf ein Enantiomeres)
(R)-(-)-Glycidylbutyrat
Reinheit: 99,64% (GC), ee 95,5% (HPLC nach Derivatisierung mit (S)-(-)-1-Phenylethylamin),
$[\alpha]_D^{25} = -31,9^\circ$

Beispiele 2 - 18

Die folgenden Beispiele wurden mit jeweils 10 ml wässriger Phase und 0,576 g (4 mmol) (R)-Glycidylbutyrat bzw. 1,15 g (4 mmol (R) + 4 mmol (S)) (RS)-Glycidylbutyrat und 50 mg Schweinepankreas-Lipase durchgeführt Die angegebenen Quotienten Q wurden auf die oben beschriebene Weise bestimmt.

| Beispiel | Fettsäurezusatz | Temp. °C kleiner als | pH | Q |
|---|---|---|---|---|
| 2 | --- | 4 | 6,0 | 4,3 |
| 3 | Na-Caprylat 0,25M | 4 | 6,5 | 11,1 |
| 4 | Na-Heptanoat 0,05M | 4 | 6,0 | 10,2 |
| 5 | Na-Capronat 0,4M | 4 | 6,0 | 11,1 |
| 6 | Caprinsäure 4mg/10ml | 4 | 5,8 | 17,0 |
| 7 | Laurinsäure 50mg/10ml | 4 | 6,3 | 11,8 |
| 8 | Oelsäure 200µl/10ml | 4 | 6,3 | 17,0 |
| 9 | Pelargonsäure 50µl/10ml | 4 | 6,3 | 11,8 |

| Beispiel | Na-Butyrat | Q |
|---|---|---|
| 10 | 0,2 M | 6,6 |
| 11 | 0,3 M | 6,7 |
| 12 | 0,4 M | 10,8 |
| 13 | 0,6 M | 11,5 |
| 14 | 0,8 M | 10,9 |
| 15 | 1,0 M | 13,9 |
| 16 | 1,2 M | 22,8 |
| 17 | 1,4 M | 20,4 |
| 18 | 1,6 M | 17,0 |

Beispiele 19 und 20

Die folgenden Beispiele wurden wie für die Beispiele 2 - 18 beschrieben durchgeführt, jedoch mit jeweils 10 mg Enzym.

| Beispiel | Enzym | Q ohne Zusatz | Q mit 0,04 M Natriumcapronat |
|---|---|---|---|
| 19 | Mucor-Lipase | 3,2 | 7,6 |
| 20 | Amano-P-Lipase | 2,7 | 6,6 |

## Ansprüche

1. Verfahren zur Herstellung von (R)-Glycidylestern von n-Carbonsäuren mit 2 - 6 C-Atomen, dadurch gekennzeichnet, dass von den entsprechenden racemischen Glycidylestern die (S)-Enantiomeren durch eine Lipase aus der Gruppe Schweinepankreas-Lipase, Mucor-Lipase und Amano-P-Lipase in Gegenwart von 0,01 bis 2,0 mol/l von Fettsäuren mit 4 - 24 C-Atomen und/oder deren wasserlöslichen Salzen enantioselektiv hydrolysiert werden.

2. Verfahren nach Anspruch 1, dadurch gekennzeichnet, dass die Hydrolyse in einem Zweiphasensysyem aus Glycidylester und wässriger Enzymlösung ohne zusätzliches Lösungsmittel durchgeführt wird.

3, Verfahren nach einem oder beiden der Ansprüche 1 und 2, dadurch gekennzeichnet, dass die Hydrolyse bei einem pH-Wert der wässrigen Phase von 5 bis 8 durchgefuhrt wird.

4. Verfahren nach einem oder mehreren der Anspruche 1 bis 3, dadurch gekennzeichnet, dass die Hydrolyse bis zu einem Gesamtumsatz von 50 bis 70% durchgeführt wird.

5. Verfahren nach einem oder mehreren der Ansprüche 1 bis 4, dadurch gekennzeichnet, dass die Hydrolyse bei einer Temperatur von 0 bis 20 °C durchgeführt wird.

6. Verfahren nach einem oder mehreren der Ansprüche 1 bis 5, dadurch gekennzeichnet, dass als Lipase Schweinepankreas-Lipase eingesetzt wird.

7. Verfahren nach einem oder mehreren der Ansprüche 1 bis 6, dadurch gekennzeichnet, dass als Glycidylester Glycidylbutyrat eingesetzt wird.

8. Verfahren nach einem oder mehreren der Ansprüche 1 bis 7, dadurch gekennzeichnet, dass als Fettsäure Oelsäure eingesetzt wird.